# EUROPEAN PATENT SPECIFICATION

(11) **EP 1 878 394 B1**
(45) Date of publication and mention of the grant of the patent: **07.09.2016**
(21) Application number: 07252513.2
(22) Date of filing: 21.06.2007
(51) Int. Cl.: A61B 17/17, A61B 17/80

(54) **Orthopaedic fixation plate having threaded guides**
Orthopädische Befestigungsplatte mit einschraubbarer Bohrerführung
Plaque de fixation orthopédique avec guide filetée

(30) Priority: 13.07.2006 US 457305
(43) Date of publication of application: 16.01.2008
(73) Proprietor: Biomet C.V., Gibraltar (GI)
(72) Inventor: Castaneda, Javier E., Miami, FL 33186 (US); Bremer, Chris, Warsaw, IN 46582 (US); Donnelly, Steve, Willoughby, OH 44094 (US); Guzman, Pam, Ft Wayne, IN 46804 (US); Stoller, Dennis, Ft Wayne, IN 46835 (US)
(74) Representative: Mays, Julie

(56) References cited:
- WO-A-99/05968
- WO-A-2006/065512
- US-A- 5 474 553
- US-A1- 2002 045 897

## Description

This invention relates broadly to surgery and, more specifically, to orthopaedic fixation plate systems. More particularly, this invention relates to guides which can be releasibly coupled to orthopaedic fixation plates.

Orthopaedic surgery to repair fractures is challenging. Proximal humeral fractures are no different in that regard. As the fracture site is prepared to receive a bone plate, the surgeon must handle numerous instruments and then properly locate the implant in the desired fixation location and temporally secure it for evaluation before permanently securing the implant to bone. In placing the implant, there is no defined manner of handling the plate securely while manipulating it among the skin, fat, and muscle of the patient to its desired location easily. Furthermore, the available plates include limited locations for placement of K-wires which are used for temporarily securing the implant against the bone. Often, there is a desirable location for temporarily anchoring the plate but such location cannot be used due to the location of a screw hole of unsuitable dimension for the K-wire.

In addition, after evaluative placement when it is necessary to permanently secure the plate with screws, the surgeon may encounter difficulty using the currently available drill guides to drill holes for the screw. For example, polyaxial bushings provided in holes of a plate are disclosed in US-5954722 and US-A-2005/0049594. Document US-A-5954722 shows a fixation plate system. The systems are intended for use with an approximately 152 mm (6 inch) long drill guide that may be screwed into a bushing and which allows the surgeon to drill a hole in the bone along the polyaxial bushing axis without injuring surrounding tissue. Unfortunately, due to limited access and visibility of the plating site, the surgeon can encounter difficulty screwing the taper threaded, distal end of the drill guide into the polyaxial bushing, especially when the plate is already positioned and/or attached to the bone. This may lead to damage of the bushing threads due to cross threading, which can potentially impair the function of the bushing. Furthermore, the time required to use the drill guide for drilling a hole through each of the many polyaxial bushings may be significant, thereby lengthening the time of the surgical procedure. Morever, the surgical step of drilling occurs during a crucial phase of the procedure when it is very important to maintain the alignment and reduction of bone fragments while fixation of the fragments is achieved. In addition, extensive fluoroscopy is often used during surgery to direct the bone drill into each of the screw holes to ensure proper alignment. Typically several images are obtained for both the lateral and anterior-posterior views, resulting in significant fluoroscopy time for each hole drilled. Where several holes are drilled, the accumulated time under fluoroscopy and associated radiation exposure can be high.

The present invention provides a fixation plate system according to claim 1. Such plate preferably includes screw holes each provided with a polyaxial bushing having an internal triple lead taper thread.

The guide can be used to manipulate a plate during implantation.

The guide can provide increased visibility and access to the plate.

Preferably, the guide can temporarily convert a large diameter screw hole into a K-wire receiving hole.

Preferably, the guide can be used in a polyaxial bushing which does not require the step of coupling the guide to the plate during the surgical procedure.

Preferably, the guide can help to reduce the time taken for a surgical procedure in which a plate is implanted.

Preferably, the guide can help to reduce the radiation to which patient is exposed during a surgical procedure.

Preferably, the guide is a relatively short element including a bottom threaded portion, a top portion, and a central passage for guiding an orthopaedic device, for example, K-wire or drill, into bone. Preferably, the threaded end is preferably provided with a triple lead taper thread that matches the taper thread of the bushing. Preferably, the central passage may approximate the minor diameter of threads on a fastener used through the hole to secure the plate to bone, or may be of a smaller diameter for another orthopaedic device, for example, for stably receiving a K-wire. Preferably, the passage at the top portion is preferably provided with tool engagement structure, for example, corners for receiving a driver. Preferably, the short length of the device permits multiple guides to be pre-assembled to the plate at once without unnecessarily obscuring and crowding the area of interest. Preferably, the guides are coupled to the plate in alignment with the axes of the polyaxial bushings, and such alignment can be set by the manufacturer in accord with preferred preset axes or by a medical professional closer to the time of surgery (before or during) in accord with desired anatomical specifications of a particular procedure.

The pre-assembled guides can preferably be lightly screwed initially into respective polyaxial bushings of the plating system, permitting the surgeon to modify the angle of each guide and bushing to a selected orientation before locking the bushing at the orientation by tightening of the guide into the bushing. This tightening may occur either before or after positioning the plate on the bone. Once the bone holes have been drilled, the surgeon preferably uses a tool to remove each guide before inserting a bone screw into the bushing and drilled hole.

The top end can define a handle, preferably with external gripping structure, for example, longitudinal ridges, to facilitate manual manipulation. The guide, preferably pre-assembled to the plate, can be used to manipulate the plate on to a patient's bone.

A guide can be provided with an extension. The guide and extension are easily coupled by longitudinal engagement to provide a longer guide for axial alignment, to rotationally insert and remove the guides, and to facilitate certain procedures where a longer guide for orthopaedic instrumentation is helpful. The extension can easily be removed from the guide when a short non-obtrusive guide is desired using a quick longitudinal movement.

The guide of the invention can be used in a method of fracture fixation, comprising:
(a) providing a plate having at least one hole with a polyaxial bushing including an internal thread, and a guide pre-assembled into at least one of the at least one hole;
(b) at the option of a medical professional, modifying an axial alignment of at least one bushing by angular displacement of the guide pre-assembled thereto; and
(c) temporarily locking an axial alignment for a bushing by rotating the respective guide to tighten the guide into the bushing.

Embodiments of the invention will now be described by way of example with reference to the accompanying drawings, in which:
Fig. 1 is a longitudinal section view of a fixation plate provided with a plurality of threaded guides according to the invention;
Fig. 2 is a side elevation view of a first embodiment of a threaded guide according to the invention;
Fig. 3 is a perspective view of the threaded guide of Fig. 2;
Fig. 4 is a perspective view of a fixation plate provided with a second embodiment of a threaded guide according to the invention;
Fig. 5 is a side elevation view of the second embodiment of a threaded guide according to the invention;
Fig. 6 is a perspective view of the threaded guide of Fig. 5;
Fig. 7 is a view similar to Fig. 4 shown with a guide extension coupled to the threaded guide;
Fig. 8 is a side elevation of the guide extension of Fig. 7;
Fig. 9 is a section view along lines 9-9 in Fig. 8;
Fig. 10 is a distal end view of the guide extension of Fig. 7;
Fig. 11 is an exploded side elevation view of a third embodiment of a guide according to the invention;
Fig. 12 is an exploded perspective view of the third embodiment of a guide according to the invention;
Fig. 13 is a broken partial section view of an assembled guide according to the third embodiment of the invention;
Fig. 14 is an exploded side elevation view of a fourth embodiment of a guide according to the invention; and
Fig. 15 is an exploded perspective view of the fourth embodiment of a guide according to the invention.

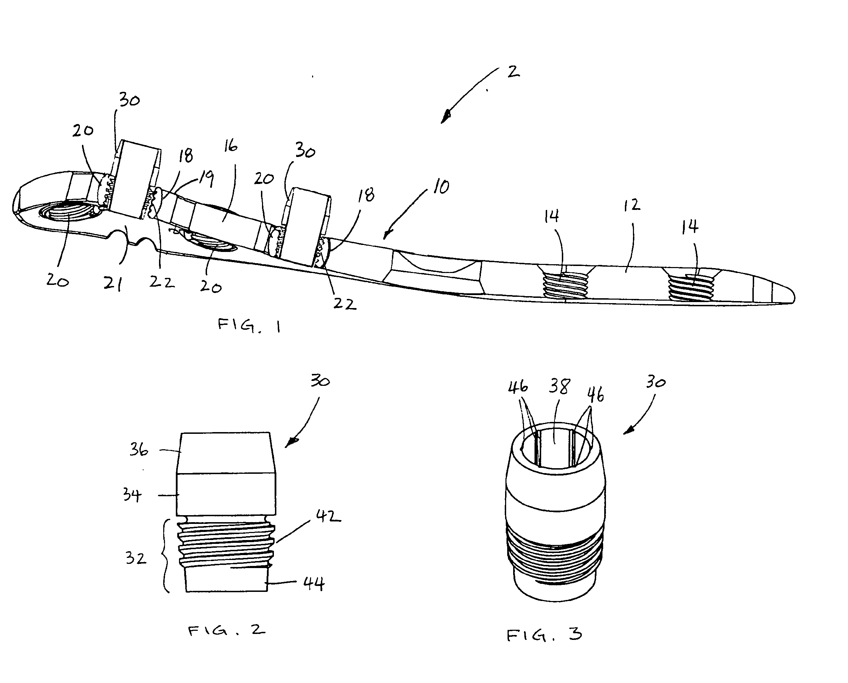

Referring to the drawings, Fig. 1 show a system 2 which includes a fixation plate 10 with guides 30 according to the invention is shown. The plate 10 includes a shaft portion 12 including threaded screw holes 14 and a head portion 16 including holes 18. The holes 18 each define a spherically curved inner surface extending between a bone contacting surface 19 and an upper (opposite) surface 21. A polyaxial bushing 20 is provided in each such hole. The interior surface of each bushing 20 includes a triple lead taper thread 22. Examples of such holes and bushings are disclosed in US-5954722 and US-A-2005/0049594. A removable guide 30 is provided for the bushings 20.

Referring to Figs. 1 through 3, according to a first embodiment of the invention, the guide 30 is a relatively short device including a bottom portion 32, a central portion 34, a top portion 36, and an axial passage 38. The bottom portion 32 is tapered with triple lead threads 42 sized for engagement with the triple lead taper threads 22 of the polyaxial bushing 20. The triple lead threads 42 preferably extend through approximately 360°. The distal end of the bottom portion 32 preferably includes a non-threaded lead-in portion 44 that facilitates insertion of the guide 30 into the bushing 20. The top portion 36 is preferably tapered to increase clearance between other guides 30 coupled to the plate 10. The axial passage 38 in the embodiment shown approximates the minor diameter of a threaded fastener to be used through the bushing 20 and is thus of suitable dimension to receive a drill bit for drilling a hole in the bone for such threaded fastener. Alternatively, the diameter may be sized substantially smaller than the hole defined by bushing 20 for stably receiving a K-wire or other temporary fastener or another orthopaedic instrument. The passage 38 at the top end is preferably provided with tool engagement structure, for example six corners 46 for receiving a hex driver. Thus, the same tool used to remove the guide (a hex driver) can be used to insert a fastener having a hex slot into the hole thereafter. The short length of the device, preferably not more than 25.4 mm (1 inch), and more preferably approximately 8.9 to 15.2 mm (0.35 to 0.6 inch), permits multiple guides 30 to be assembled to the plate 10 at once without unnecessarily obscuring and crowding the area of interest at the surgical site. The guides 30 are coupled to the plate in alignment with the axes of the polyaxial bushings, and such alignment can be set by the manufacturer in accord with preferred preset axes or by a medical professional closer to the time of surgery (before or during) in accord with desired anatomical specifications of a particular procedure. The guides are not coupled to the plate as tightly as the threaded fastener that will eventually be positioned in its place and tightened within the bushing. Therefore, the guides 30 may be used as small handles to redirect the axial alignment of the bushing 20. As discussed with respect to other embodiments below, guide extensions can be coupled to the guides to facilitate such redirection and the drilling of holes.

In a preferred use, a plate with guides pre-assembled therein in preferred axial alignments is provided to the surgeon. The pre-assembled guides are preferably lightly screwed into respective polyaxial bushings of the plating system permitting the surgeon to modify the angle of each guide and bushing to a selected orientation (angular displacement) before locking the bushing at the orientation by tightening of the guide into the bushing. This tightening may occur either before or after positioning the plate on the bone. Once the bone holes have been drilled, the surgeon preferably uses a tool, for example, a hex driver, or a guide extension, discussed below, to remove each guide before inserting a fastener into the bushing and drilled hole.

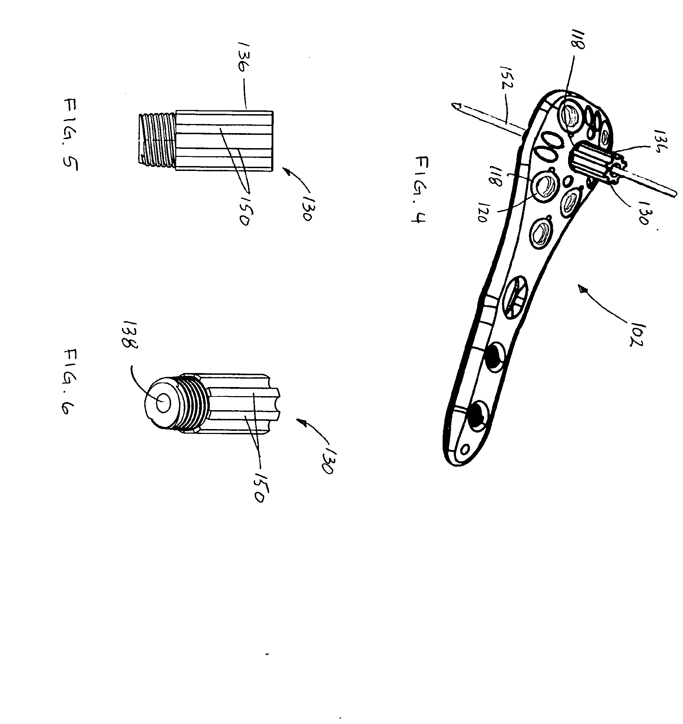

Figs. 4 to 6 show a second embodiment of the system 102 in which the guide 130 has a top end defining a handle 136, preferably with external gripping structure, for example, longitudinal ridges 150, to facilitate manual manipulation of the axial orientation of bushing or of the plate as whole. In addition, the passage 138 through the guide in the embodiment shown is sized for a K-wire 152. Thus, a screw hole 118 and bushing 120 that under normal circumstances would be too large for stable fixation by K-wire can be used for temporary fixation of the implant on the bone with K-wire.

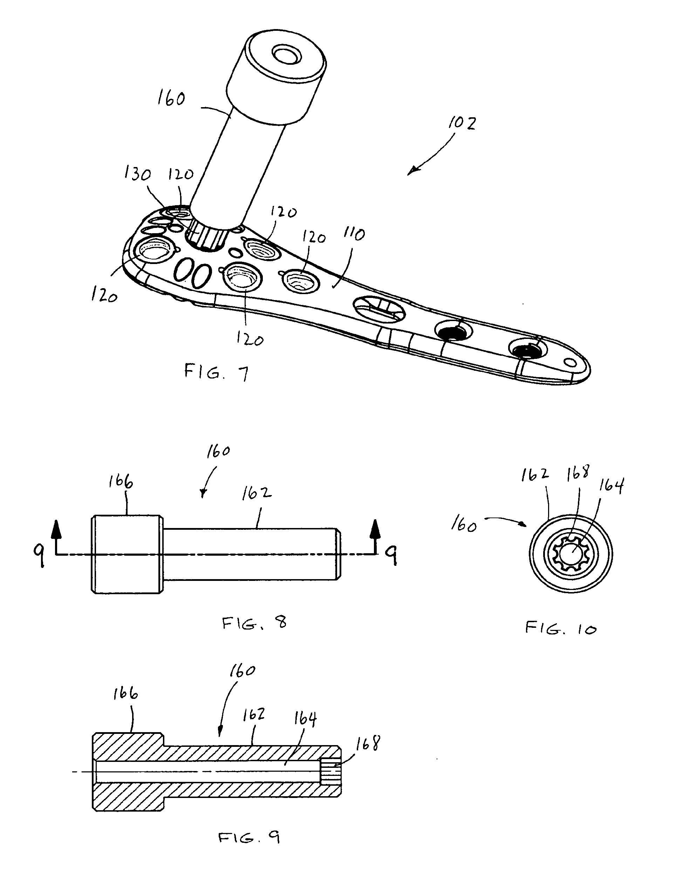

Figs. 7 to 10 show the system 102 with a guide extension 160 coupled to the guide 130 in the plate 110. The guide extension 160 includes a shaft 162 defining a central passage 164, a proximal handle 166, and a distal socket 168 for engagement over the top end of the guide 130. When positioned over the guide, the central passages 138, 168 are aligned and preferably of the same dimension so as to provide a composite guiding passageway of longer length for a K-wire or orthopaedic device for which the passageway is sized. The guide extension 160 is rotationally fixed relative to the guide 130 so as to also facilitate insertion of the guide 130 into the bushing 120, axial alignment of the guide 130 and bushing 120 relative to the plate 110, tightening of the guide 130 relative to the bushing 120, and removal of the guide 130 from the bushing 120.

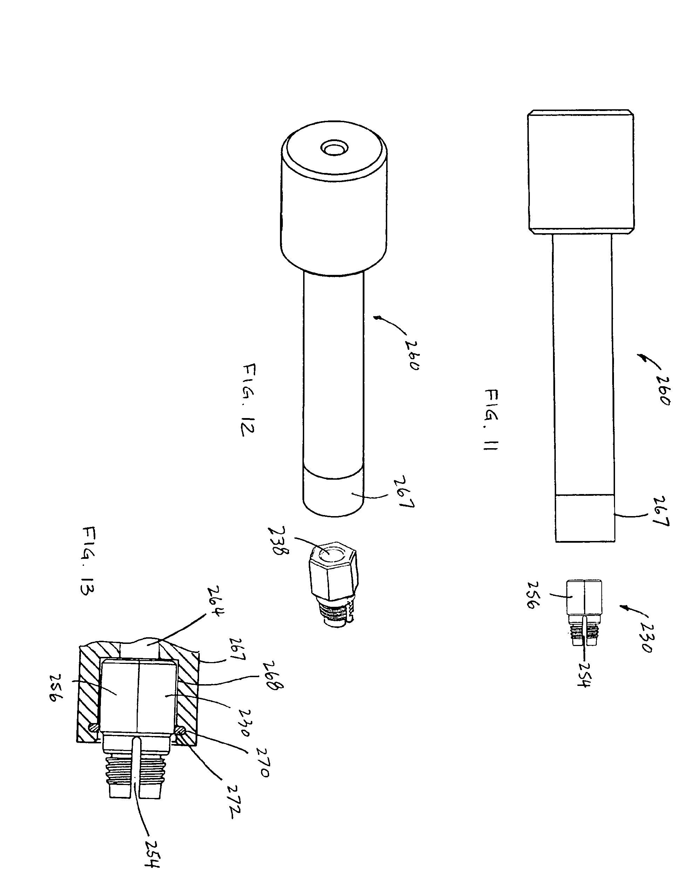

Figs. 11 to 13 show a third embodiment of a guide 230 and guide extension 260. The guide 230 is similar to the above in that it is preferably substantially short, provided pre-assembled to the plate, and includes a central passageway for, for example, K-wire or instrument guidance. The distal portion of the guide 230 includes a diametric slot 254. In the event the guide 230 is inserted with too much force into the bushing 20 (for example, Fig. 1), the slot 254 allows compression of such distal portion of the guide 230 within the bushing so as to prevent cross threading. The proximal portion of the guide 230 includes a non-circular head 256, preferably hexagonal in shape, for engagement by the extension 260. The distal end 267 of the extension 260 includes socket 268 that captures at least a portion of the proximal portion of the guide 230 in a rotationally interfering manner. Preferably, the distal socket 268 has a corresponding shape, for example, hex socket, to the proximal portion of the guide. To eliminate play in the assembly of the guide 230 and extension 260, it is preferred to provide a groove 270 in the socket, and a resilient o-ring 272 in the groove that will contact the guide. Once so assembled, the central passages 238, 264 of the guide and extension are aligned.

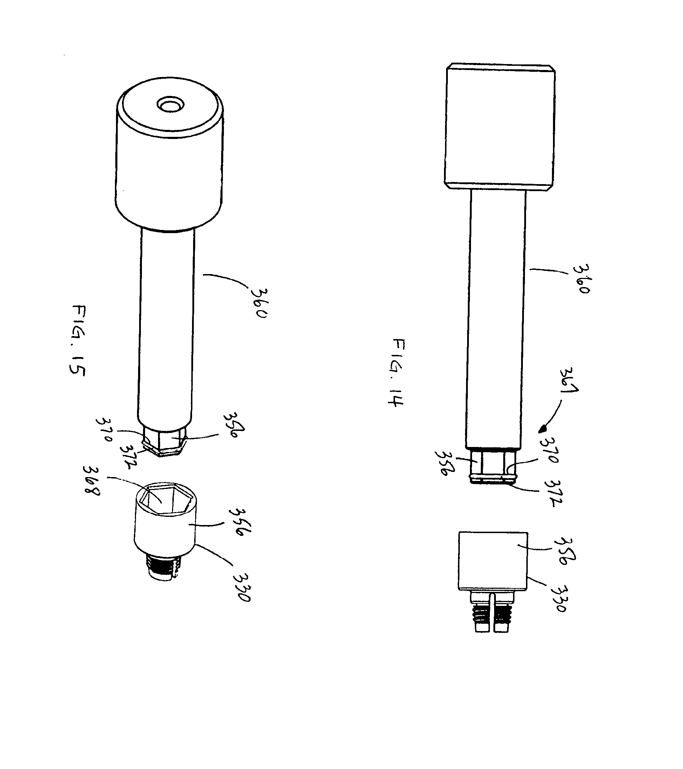

Figs. 14 and 15 show another embodiment in which a socket 368 is provided to the proximal portion of the guide 330, the distal end 367 of the extension 360 includes a head 356 which engages in a rotationally interfering manner within the socket 368, and a groove 370 and o-ring 372 are provided externally about the head 356 to reduce play in the assembly of the guide and extension.

The guide extension may be provided with a central passageway defining a diameter that is smaller than the diameter of the central passageway of the guide. Such smaller diameter of the guide extension may, for example, correspond to the diameter of a K-wire, while the diameter of the guide corresponds to a drill for the locking bone fastener. Using the guide and guide extension, the surgeon can first drill a pilot hole with a K-wire and once satisfied with its location within the bone, then remove the extension and drill through the guide with the regular drill bit for the locking bone fastener.

## Claims

1. An orthopaedic fixation plate system (2), comprising:
a fixation plate (10) including a bone contacting surface (19) and an opposite surface (21), and a hole (18) extending between the surfaces, the hole (18) being spherically curved between the surfaces and the system (2) further comprising:
a polyaxial bushing (20) provided in the hole (18); and
a removable guide (30) coupled in the polyaxial bushing (20), the guide (30) having a top portion (36), a bottom portion (32), and a central passageway (38), **characterised in that** the guide (30) has a length of not more than about 25 mm (1 inch), and **in that** the polyaxial bushing (20) can be locked in a fixed orientation relative to the plate (10).

2. A system (2) according to claim 1, in which the bushing (20) and the bottom portion (32) of the guide (30) each include tapered threads (22, 42).

3. A system (2) according to claim 1, in which the bushing (20) and the bottom portion (32) of the guide (30) each include a triple lead thread (22, 42).

4. A system (2) according to claim 1, in which the guide (30) has a length of 8.9 to 15.2 mm (0.35 to 0.6 inch).

5. A system (2) according to claim 1, in which the plate (10) has at least two holes (18), each provided with a polyaxial bushing (20), and a removable guide (30) being coupled to each polyaxial bushing (20).

6. A system (2) according to claim 1, in which the bottom portion (32) of the guide (30) includes a distal portion (44) that is non-threaded.

7. A system (2) according to claim 1, in which the top portion (36) of the guide (30) is tapered.

8. A system (2) according to claim 1, in which the central passage (38) is substantially smaller than an inner diameter of the bushing (20).

9. A system (2) according to claim 1, in which the top portion (36) of the guide (30) includes structure (46) that permits rotational interference with the guide (30) by a tool.

10. A system (2) according to claim 1, in which the guide (30) includes external gripping structure (150) to facilitate manipulation.

11. A system (2) according to claim 1, which includes a guide extension (160, 260, 360) that is removably coupled to the guide (30), the guide extension (160, 260, 360) including a passageway (164) that is aligned with the central passageway (38) of the guide (30) when the guide (30) and guide extension (160, 260, 360) are coupled together, the guide (30) and guide extension (160, 260, 360) being coupled together in a rotationally interfering manner.

12. A system (2) according to claim 11, in which the guide extension (160, 260, 360) includes a resilient member to remove play in the coupling of the guide extension (160, 260, 360) and guide (30).

13. A system (2) according to claim 11, in which the passageway (164) of the guide extension (160, 260, 360) has a smaller diameter than the central passageway (38) of the guide (30).

## Patentansprüche

1. Orthopädisches Fixierungsplattensystem (2), umfassend:
eine Fixierungsplatte (10) mit einer Knochenberührungsoberfläche (19) und einer entgegengesetzten Oberfläche (21) und mit einem Loch (18), das sich zwischen den Oberflächen erstreckt, wobei das Loch (18) zwischen den Oberflächen sphärisch gekrümmt ist, und wobei das System (2) ferner folgendes umfasst:
eine polyaxiale Buchse (20), die in dem Loch (18) vorgesehen ist; und
eine entfernbare Führung (30), die in der polyaxialen Buchse (20) gekoppelt ist, wobei die Führung (30) einen oberen Teil (36), einen unteren Teil (32) und einen zentralen Durchgang (38) aufweist, **dadurch gekennzeichnet, dass** die Führung (30) eine Länge von nicht mehr als etwa 25 mm (1 Inch) aufweist, und wobei die polyaxiale Buchse (20) in einer ersten Ausrichtung im Verhältnis zu der Platte (10) gesperrt werden kann.

2. System (2) nach Anspruch 1, wobei die Buchse (20) und der untere Teil (32) der Führung (30) jeweils konische Gewinde (22, 42) aufweisen.

3. System (2) nach Anspruch 1, wobei die Buchse (20) und der untere Teil (32) der Führung (30) jeweils ein Dreiwegegewinde (22, 42) aufweisen.

4. System (2) nach Anspruch 1, wobei die Führung (30) eine Länge von 8,0 bis 15,2 mm (0,35 bis 0,6 Inch) aufweist.

5. System (2) nach Anspruch 1, wobei die Platte (10) wenigstens zwei Löcher (18) aufweist, die jeweils mit einer polyaxialen Buchse (20) versehen sind, und wobei eine entfernbare Führung (30) mit jeder polyaxialen Buchse (20) gekoppelt ist.

6. System (2) nach Anspruch 1, wobei der untere Teil (32) der Führung (30) einen distalen Teil (44) aufweist, der ohne Gewinde ist.

7. System (2) nach Anspruch 1, wobei der obere Teil (36) der Führung (30) konisch ist.

8. System (2) nach Anspruch 1, wobei der zentrale Durchgang (38) im Wesentlichen kleiner ist als ein Innendurchmesser der Buchse (20).

9. System (2) nach Anspruch 1, wobei der obere Teil (36) der Führung (30) eine Struktur (46) aufweist, die einen Dreheingriff mit der Führung (30) durch ein Instrument erlaubt.

10. System (2) nach Anspruch 1, wobei die Führung (30) eine externe Greifstruktur (150) aufweist, um die Manipulation zu erleichtern.

11. System (2) nach Anspruch 1, wobei dieses eine Führungsverlängerung (160, 260, 360) aufweist, die entfernbar mit der Führung (30) gekoppelt ist, wobei die Führungsverlängerung (160, 260, 360) einen Durchgang (164) aufweist, der mit dem zentralen Durchgang (38) der Führung (30) ausgerichtet ist, wenn die Führung (30) und die Führungsverlängerung (160, 260, 360) miteinander gekoppelt sind, wobei die Führung (30) und die Führungsverlängerung (160, 260, 360) durch Dreheingriff miteinander gekoppelt werden.

12. System (2) nach Anspruch 11, wobei die Führungsverlängerung (160, 260, 360) ein elastisches Element aufweist, um das Spiel in der Kopplung zwischen der Führungsverlängerung (160, 260, 360) und der Führung (30) zu entfernen.

13. System (2) nach Anspruch 11, wobei der Durchgang (164) der Führungsverlängerung (160, 260, 360) einen kleineren Durchmesser aufweist als der zentrale Durchgang (38) der Führung (30).

## Revendications

1. Système de plaque de fixation orthopédique (2), comprenant :
une plaque de fixation (10) comprenant une surface (19) en contact avec l'os et une surface opposée (21), et un orifice (18) s'étendant entre les surfaces, l'orifice (18) étant sphériquement courbé entre les surfaces, et le système (2) comprenant en outre :
une douille polyaxiale (20) disposée dans l'orifice (18) ; et
un guide amovible (30) accouplé dans la douille polyaxiale (20), le guide (30) ayant une partie supérieure (36), une partie inférieure (32) et un passage central (38), **caractérisé en ce que** le guide (30) a une longueur inférieure ou égale à environ 25 mm (1 po), et **en ce que** la douille polyaxiale (20) peut être verrouillée dans une orientation fixe par rapport à la plaque (10).

2. Système (2) selon la revendication 1, la douille (20) et la partie inférieure (32) du guide (30) comprenant chacune des filetages coniques (22, 42).

3. Système (2) selon la revendication 1, la douille (20) et la partie inférieure (32) du guide (30) comprenant chacune un fil de plomb triple (22, 42).

4. Système (2) selon la revendication 1, le guide (30) ayant une longueur comprise entre 8,9 et 15,2 mm (entre 0,35 et 0,6 pouce).

5. Système (2) selon la revendication 1, la plaque (10) ayant au moins deux orifices (18), tous deux comprenant une douille polyaxiale (20), et un guide amovible (30) étant accouplé à chaque douille polyaxiale (20).

6. Système (2) selon la revendication 1, la partie inférieure (32) du guide (30) comprenant une partie distale (44) qui n'est pas filetée.

7. Système (2) selon la revendication 1, la partie supérieure (36) du guide (30) étant conique.

8. Système (2) selon la revendication 1, le passage central (38) étant sensiblement plus petit que le diamètre interne de la douille (20).

9. Système (2) selon la revendication 1, la partie supérieure (36) du guide (30) comprenant une structure (46) qui permet une interférence rotative avec le guide (30) par un outil.

10. Système (2) selon la revendication 1, le guide (30) comprenant une structure de préhension externe (150) pour faciliter la manipulation.

11. Système (2) selon la revendication 1, qui comprend une extension de guide (160, 260, 360) qui est accouplée amovible au guide (30), l'extension de guide (160, 260, 360) comprenant un passage (164) qui est aligné avec le passage central (38) du guide (30) lorsque le guide (30) et l'extension de guide (160, 260, 360) sont accouplés, le guide (30) et l'extension de guide (160, 260, 360) étant accouplés de sorte à permettre une interférance rotative.

12. Système (2) selon la revendication 11, l'extension de guide (160, 260, 360) comprenant un élément résilient pour supprimer le jeu dans l'accouplement de l'extension de guide (160, 260, 360) et du guide (30).

13. Système (2) selon la revendication 11, le passage (164) de l'extension de guide (160, 260, 360) ayant un diamètre plus petit que le passage central (38) du guide (30).
